Europäisches Patentamt

European Patent Office

Office européen des brevets

(19)

(11) **EP 1 584 687 A1**

(12) **EUROPEAN PATENT APPLICATION**

(43) Date of publication:
**12.10.2005 Bulletin 2005/41**

(51) Int Cl.$^7$: **C12N 15/69**

(21) Application number: **04008557.3**

(22) Date of filing: **08.04.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(71) Applicant: **Boehringer Ingelheim Austria GmbH
1120 Wien (AT)**

(72) Inventors:
• **Huber, Hans
1020 Wien (AT)**

• **Pacher, Claudia
2380 Perchtoldsdorf (AT)**
• **Necina, Roman
1210 Wien (AT)**
• **Kollmann, Franz
4020 Linz (AT)**
• **Reinisch, Christoph
1120 Wien (AT)**

(74) Representative: **Hammann, Heinz et al
c/o Boehringer Ingelheim GmbH,
Postfach 200
55216 Ingelheim am Rhein (DE)**

(54) **Method for producing plasmid DNA on a manufacturing scale by fermentation of the Escherichia Coli K-12 strain JM108**

(57)     A method for producing plasmid DNA on a manufacturing scale uses *Escherichia coli* K-12 strain JM108. The process results in high yield and homogeneity of plasmid DNA.

EP 1 584 687 A1

## Description

### Field of the invention

This invention refers to fermentation of *Escherichia coli* cells for the production of plasmid DNA (pDNA), in particular for use in gene therapy and DNA vaccination.

### Introduction

The requirement for fermentation of pDNA on a manufacturing scale has come up due to the clinical success of gene therapy and DNA vaccination during the last decade.

Gene therapy is the treatment or prevention of disease by the administration, delivery and expression of genes in mammalian cells. The ultimate goal of gene therapy is to cure both inherited and acquired disorders by adding, correcting, or replacing genes. Basically, there are two types of gene therapy vectors to achieve these goals, i.e. viral vectors based on inactivated viruses and non-viral vectors based on plasmid DNA. The present invention relates to the production of non-viral plasmid DNA.

Since it was demonstrated that intramuscular injection of pDNA encoding an antigen elicits both a humoral and a cellular immune response, naked plasmid DNA has become of particular importance.

The efficiency of a fermentation process for manufacturing plasmid DNA is characterized by a high yield of pDNA, either per volume fermentation broth (volumetric yield) or per biomass aliquot (specific yield). In the meaning of the present invention, yield is the concentration of plasmid DNA per volume or cell weight. Beyond a high yield, the plasmid has to be present in its intact covalently closed circular (ccc) or supercoiled form. In the meaning of the invention, the percentage of ccc form is termed "plasmid homogeneity". The concentration of other plasmid forms such as open circular (oc), linear and dimeric or multimeric forms, should be reduced to a minimum in the purified plasmid bulk, and are consequently not desired during fermentation.

Therapeutic plasmids consist of three essential parts, i.e. the therapeutic gene (the "gene of interest") under the control of a eukaryotic promoter, mostly the cytomegalovirus (CMV) promoter, an origin of replication (*ori*) for the autonomous propagation in the prokaryotic cell, and a selection marker, usually an antibiotic resistance gene. The therapeutic gene is selected in view of its clinical and medicinal relevance, while both the *ori* and the selection marker play a crucial role in plasmid production, especially during fermentation. For constructing a therapeutic plasmid, a key factor is the choice of an origin of replication that replicates to a high number of plasmid copies per cell. Most therapeutic vectors bear the ColE1-type ori. Plasmids having a ColE1 origin derived from pBR322 may reach copy numbers of 50-100 plasmids per cell; plasmids derived from pUC can reach copy numbers of several hundred.

The antibiotic selection marker and the use of antibiotics are necessary during transformation and selection of plasmid harboring cells. However, antibiotic selection pressure should be avoided during industrial manufacturing. It is therefore desirable to develop fermentation processes allowing a stable propagation of the vector without plasmid loss.

### Background of the invention

The choice of the bacterial host strain has been shown to be a key factor that needs to be considered for fermentation of pDNA. Desirable host phenotypes have the ability to grow to a high cell density, to achieve high plasmid copy numbers, to generate a minimum of plasmid-free cells, to have a minimum potential for genetic alterations of the plasmid, to produce plasmids that are predominantly supercoiled, and to be compatible with common purification procedures.

Although most strains of E. *coli* are, in principle, suitable for propagating pDNA, the choice of the specific host strain has been shown to have significant impact on the yield and quality of the recovered plasmids (Schoenfeld et al., 1995; Schleef, 1999). Currently, there is no consensus on the genotypic or phenotypic characteristics that would be ideal for bacterial strains used for manufacturing pDNA; there is little published work on optimizing host strains for the purpose of pDNA manufacturing and no data are available from comparative studies that would show the behavior of different strains in controlled fermentations. Since the properties of a host strain sometimes depend on the plasmid of interest, empirical evaluation of several hosts may, when using different plasmids, give substantially different results (Durland and Eastman, 1998).

The phenotype that a strain shows is the result of its particular genotype, i.e. the mutations of specific genes it has. A number of genetic mutations have been suggested that may have an impact on manufacturing of pDNA (Durland and Eastman, 1998). Desirable genotypes include markers that ensure the structural integrity of the plasmid or such which increase the yield due to enhanced plasmid replication.

*E. coli* DH5α is a frequently used strain or fermentation of plasmid DNA (O'Kennedy et al., 2003; WO 02/064752; O'Kennedy et al., 2000; WO 99/61633). With this strain, high volumetric yields of pDNA (ranging between 70 and 230 mg/L) with a high homogeneity (> 90 % supercoiled) were obtained (WO 02/064752; WO 99/61633). However, O'Kennedy et al. (2003) showed that, depending on the fermentation strategy, pDNA isolated from DH5$\alpha$ was consistently only 50 to 70 % supercoiled. A low degree of superhelicity was also observed with the strain DH10B. Although a high pDNA yield between 100 and 220 mg/L was shown in fed-batch fermentation, the formation of open circular or concatenated (dimeric, multimeric) plasmid forms are reported for plasmid DNA obtained from *E. coli* DH10B (Lahijani et al., 1996; Chen et al., 1997).

Other strains have been investigated in view of increasing the yield of pDNA, however, without being considered for manufacturing of plasmid DNA for therapeutic use (such as *E. coli* CP79 and CP143 (Hofmann et al., 1990) or *E. coli* HB101 (Reinikainen, 1989).

Since regulatory authorities demand supercoiled pDNA for consistency and therapeutic reasons (CBER (1996); WHO (1998); EMEA (2001)), a host that produces a substantial amount of plasmid other than in its supercoiled form is disadvantageous.

A further important issue in fermentation of plasmid DNA, which is influenced by the host strain, is the content of pDNA in the harvested biomass, i. e. the specific pDNA yield. Typical specific plasmid yields according to the current state of the art range between 2 and 10 mg per gram dry cell weight. Since buffer volumes for alkaline lysis are based on a certain biomass aliquot, a high specific pDNA yield is particularly advantageous during purification of pDNA - a high specific pDNA concentration will require lower buffer volumes and result in increased pDNA concentrations in the lysis bulk and shorter process times.

Consequently, there is a need for identifying bacterial hosts that have the ideal properties for pDNA production.

## Brief Description of the Invention

The invention relates to the use of *Escherichia coli* K-12 strain JM108 (in the following termed "JM108") or a derivative of this strain as a host strain for the fermentation of therapeutic plasmid DNA on a manufacturing-scale.

The advantageous properties of JM108 are independent of the applied fermentation process. In the experiments of the invention, the superiority of JM108 was shown for different fermentation modes (batch and fed-batch fermentation; use of defined and semi-defined medium).

The use of JM108 in manufacturing of pDNA is not restricted to a certain plasmid type, it refers to any plasmid. Preferably, plasmids with a pUC origin of replication are considered.

## Detailed Description of the Invention

*E. coli* strain K-12 JM108 is a direct progeny of *E. coli* JM106, which is derived from the strain DH1. JM108 was originally intended as a host for cosmid libraries (Yanisch-Perron et al., 1985). JM108 has the following genetic markers (i. e. specific mutations): F⁻, r*ec*A1, *end*A1, *gyr*A96, *thi*-1, hsdR-17, supE44, *rel*A1, λ⁻ and Δ(*lac-proAB*) (Yanisch-Perron et al., 1985).

Thus, JM108 has a defect in the recombination protein RecA and lacks the restriction enzyme endonuclease A (EndA), as well as a part of the type III restriction-modification system (HsdR). Furthermore, JM108 has a mutation in the *relA* gene. This gene is responsible for the synthesis of guanosine tetraphosphate (ppGpp), a signal molecule which triggers the so-called stringent response in the cell upon amino acid limitation. The genetic marker *gyrA*96 represents a mutation in the DNA-gyrase (topoisomerase II), an enzyme which influences the supercoiling state of DNA.

Since its generation, JM108 has only rarely been used for molecular biology purposes. Its applications were in recombinant protein expression (Herman and McKay, 1986; Riegert et al., 1998), preparation of cosmid libraries (Kakinuma et al., 1991; Omura et al., 2001) and transposon-facilitated DNA sequencing (Ikeda et al., 1999). In a laboratory-scale comparison of ten E. *coli* strains, no obvious superiority of JM108 was observed (Schleef, 1999).

In the experiments of the invention, JM108 was tested, together with other candidate strains, as a host strain for pDNA production. It was surprisingly found that JM108 has the ability to produce an extraordinarily high yield of pDNA, both in terms of volume and specificity. A special feature of JM108, as compared to other strains, is that plasmids in the bacterial biomass accumulate at a much higher rate. In addition, plasmid homogeneity (% ccc, supercoiled) is consistently high at more than 90 % of the ccc form. Furthermore, degradation of ccc pDNA does not occur towards the end of fermentation, as it is the case with strains used in the art. These surprising properties make JM108 an ideal host for pDNA manufacturing, which is superior in its performance compared to state of the art host strains.

Since the mutations of JM108 are not unique for JM108 (for instance, it shares the markers *recA, endA, gyrA,* and *relA* with the strains DH1, DH5, DH5$\alpha$ and XL1-blue, and, additionally, the marker *hsdR* with DH1, DH5 and DH5$\alpha$), the performance of JM108 cannot be predicted or explained simply by its mutations.

The present invention relates to a method for producing plasmid DNA on a manufacturing scale by fermenting *Escherichia coli* cells that contain a plasmid carrying a gene of interest, wherein said cells are cells of the *Escherichia*

*coli* K-12 strain JM108.

According to the invention, the term "fermentation" relates to the cultivation in a controlled industrial fermenter (bioreactor) according to methods known in the biopharmaceutical industry. The use of JM108 in fermentation to produce large amounts plasmid DNA results in a performance that is superior to the use of current state-of-the-art host strains of *E. coli.* Manufacturing of pDNA is typically accomplished by performing fermentations in large volumes. The term "manufacturing" and "manufacturing scale" in the meaning of the invention defines a fermentation with a minimum volume of 5 L culture broth.

It was found that JM108 consistently showed both a volumetric and specific pDNA yield that exceeded the results reported from fermentations of other strains.

The high specific pDNA yield obtained by the method of the invention is not only beneficial for fermentation productivity, but also for the subsequent alkaline cell lysis. The reason for this is that buffer volumes for alkaline lysis are always based on a biomass aliquot. A high plasmid content in the biomass (i. e. less biomass with the same plasmid amount) therefore results in a higher plasmid concentration of the alkaline lysate and reduced bulk volumes.

Furthermore, the homogeneity of plasmids obtained from fermentations using JM108 was found to be outstandingly high, with a yield of $\geq 90$ % in the supercoiled form. The plasmid showed no or only a weak tendency to decrease towards the end of fermentation. These surprising findings make JM108 an ideal host strain for industrial pDNA production.

In the meaning of the invention, the use of JM108 includes the use of any *E. coli* strain that is derived from JM108, i.e. any strain that has the mutations F$^-$, *recA*1*, endA*1*, gyrA*96*, thi*-1*, hsdR*-17*, supE*44*, relA*1*,* $\lambda^-$ and $\Delta$*(lac-proAB).* A derivative of JM108 may have one or more additional defined or undefined mutations, e.g. a complete or partial deletion of one or more genes, and/or carry one or more additional genes.

The application of *E. coli* JM108 for pDNA production is not restricted to the fermentation mode. The fermentation mode may be either a batch process or a fed-batch process. A batch process is a fermentation mode in which all the nutrients necessary for cultivation of the cells are contained in the culture medium, with no additional supply with nutrients during fermentation. Examples for batch fermentation processes which are useful in the invention, are given in Lahijani et al., 1996; O'Kennedy et al., 2003; and WO 02/064752. In such batch processes, plasmid-harboring *E. coli* cells (DH5$\alpha$ or DH10B), were cultivated in controlled fermenters until a certain biomass level was reached, ranging from 2.6 to 22 g dry cell weight per liter. The applied culture media were either chemically defined (WO 02/064752) or semi-defined (Lahijani et al., 1996; O'Kennedy et al., 2003), but no additional nutrient compounds were fed during fermentation time. The plasmid yield of said batch processes ranged from 8.5 to 68 mg/L.

Apart from batch processes, the method of the invention may be a fed-batch process, in which, after a batch cultivation phase, additional nutrients are supplied (fed) to the culture to obtain a higher biomass. The fed-batch process is not restricted to a particular feeding mode. Feeding of nutrients may be done in a continuous or discontinuous mode. The feeding mode may be pre-defined following a time profile, e.g. linear constant, linear increasing, step-wise increasing or exponential (Lahijani et al., 1996; O'Kennedy et al., 2003; WO 96/40905). In another embodiment of the invention, a feedback algorithm may be applied, for instance dependent on biomass, dissolved oxygen (WO 99/61633) or on pH and dissolved oxygen (Chen et al., 1997).

The application of *E. coli* JM108 for pDNA production is not restricted to the type of the culture medium of the fermentation. The culture medium may be semi-defined, containing complex media compounds (e.g. yeast extract, soy peptone, casamino acids) or may be chemically defined, without complex compounds.

The use of *E. coli* JM108 is neither restricted to a certain plasmid or plasmid type, nor to the purpose of the plasmid. In a preferred embodiment, the plasmids produced by fermenting JM108 are intended for the use in gene therapy or DNA vaccination. Plasmids suitable for application in the sense of the invention are for example derived from pBR322, pUC18, pUC19, pcDNA3™ (Invitrogen).

In a preferred embodiment of the invention, a plasmid having a ColEI origin of replication is used. The advantage of ColE1 plasmids is, that upon amino acid limitation, uncharged transfer RNAs (tRNAs) interact with the ColE1 replication origin such that an enhanced replication rate of the plasmid is the consequence (Wrobel and Wergrzyn, 1998).

In a preferred embodiment of the invention, a ColE1-derived plasmid of the pUC type (Yanisch-Perron et al., 1985) is used. The pUC plasmids have a mutation in the copy number-decreasing protein Rom, and have therefore a higher plasmid copy number than the original ColE1 type. Since the replication-enhancing effect of rom- plasmids is especially shown at low specific growth rates (Atlung et al., 1999), the combination of pUC plasmids with a low growth rate cultivation is preferred in the invention.

Plasmid DNA obtained according to the method of the invention by cultivation of *E. coli* JM 108 is recovered and purified according to known methods. Plasmid purification typically starts with the disintegration of the harvested cell mass, usually by alkaline lysis. Thereby, cells are subjected to high alkaline pH values together with detergents, so that the cells are lysed and plasmids are released. Upon the following precipitation step with acetate buffer, proteins and genomic DNA get precipitated, whereas the plasmid DNA remains in the clarified supernatant. The subsequent purification steps comprise mainly filtration (ultrafiltration, diafiltration) and chromatographic techniques. The chromatographic methods may be selected from, for example, hydrophobic interaction, ion exchange or gel filtration chromatography.

**Brief Description of the Figures**

Figure 1: Specific pDNA yields of several strains carrying different plasmids, obtained from a typical shake flask cultivation (LB-medium).

Figure 2: Plasmid homogeneity (% of ccc form) of several strains carrying different plasmids, obtained from a typical shake flask cultivation (LB-medium).

Figure 3: Time course of the specific pDNA yields of several host strains harboring the plasmid pRZ-hMCP1 in batch fermentations (semi-defined medium).

Figure 4: Plasmid and growth parameters of a batch fermentation of *E. coli* JM108 (pRZ-hMCP1) on a manufacturing scale of 200 L (semi-defined medium).

Figure 5: Analytical HPLC chromatogram of a plasmid sample obtained from the end of a batch fermentation of E. *coli* JM108 (pRZ-hMCP1) on a manufacturing scale of 200 L (semi-defined medium).

Figure 6: Plasmid and growth parameters of a batch fermentation of *E. coli* JM108 (pRZ-hMCP1) on a 1 L scale in a defined medium.

Figure 7: Analytical HPLC chromatogram of a plasmid sample obtained from the end of a batch fermentation of E. *coli* JM108 (pRZ-hMCP1) on a 1 L scale in a defined medium.

Figure 8: Plasmid and growth parameters of a fed-batch fermentation of *E. coli* JM108 (pRZ-hMCP1) on a manufacturing scale of 20 L (semi-defined medium).

Figure 9: Time course of the pDNA homogeneity of a fed-batch fermentation of *E. coli* JM108 (pRZ-hMCP1) on a manufacturing scale of 20 L (semi-defined medium).

Figure 10: Plasmid and growth parameters of a fed-batch fermentation of *E. coli* JM1 08 (pRZ-hMCP1) on a manufacturing scale of 20 L (defined medium).

Examples

**Preliminary Experiment 1**

Shake flask cultivation of different *E. coli* host strains harboring three different plasmids

In this experiment, several plasmid bearing host strains were compared in a typical lab-scale cultivation. In 100 mL baffled shake flasks containing 40 mL modified LB medium (10 g/L soy peptone; 5 g/L yeast extract; 10 g/L NaCl), seven different plasmid harboring E. *coli* strains were cultivated until early stationary phase (37 °C, rotary shaker 300 rpm). The strains harbored the therapeutic plasmids pRZ-hMCP1 (4.9 kb, coding for human monocyte chemoattractant protein 1, (Furutani et al. (1989), pGS-hil2-tet (4.3 kb; coding for human interleukin 2) and pRZ-hGM-CSF (5.4 kb; coding for human granulocyte monocyte colony stimulating factor). The cultivations were characterized in terms of growth (optical density, $OD_{550}$), volumetric plasmid yield (mg pDNA/L), specific plasmid yield (mg/OD*L) and plasmid homogeneity (% supercoiled, ccc).
For pDNA analysis, cell aliquots were lysed by a modified alkaline lysis method as originally described by Birnboim and Doly (1979) and the pDNA yield and homogeneity was determined by anion exchange high performance chromatography (Tosoh Biosep DNA-NPR-DEAE column; equilibration and load with 20 mM Tris at pH 9; separation and gradient elution with 20 mM Tris containing 1 M NaCl; flow rate 0.8 mL/min at 25°C; detection of pDNA with UV diode array detector at 260 nm). This analytical method was also applied for all pDNA analysis in the other examples presented here.

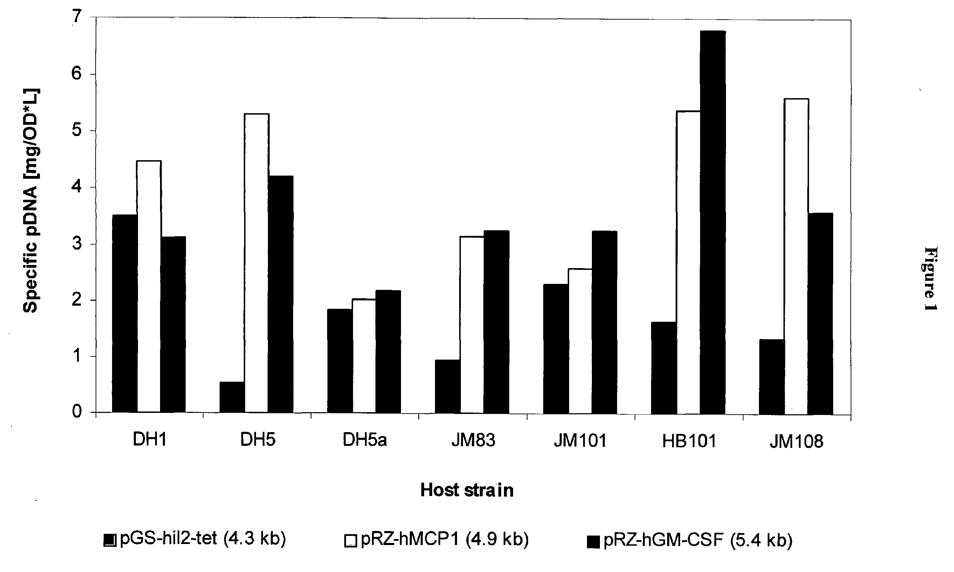

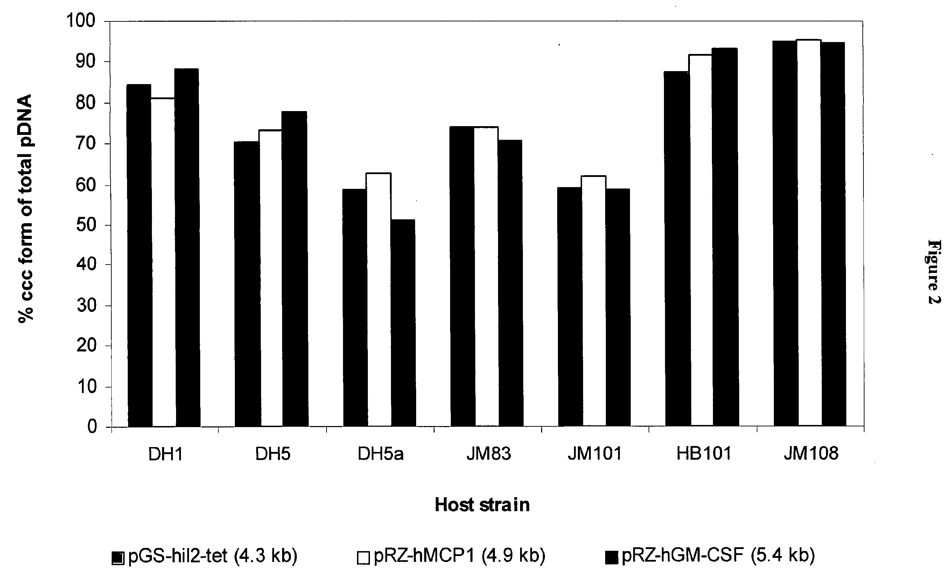

Figure 1 shows the specific pDNA of the seven strains harboring three plasmids. When cultivated in shake flasks, none of the strains showed an obvious superiority for all plasmids tested. A similar result was obtained when the volumetric pDNA yields were compared. This example demonstrates that the performance of the strain JM108 in terms of pDNA yield was not obvious when cultivated in a standard shake flask method. However, the pDNA homogeneity of JM108 was significantly higher compared to the other strains, as demonstrated in Figure 2.

**Preliminary Experiment 2**

Batch fermentations of different *E. coli* host strains harboring the plasmid pRZ-hMCP1 in a semi-defined medium (1L-scale)

In 1L-scale fermenters, with nine strains harboring the plasmid pRZ-hMCP1 (4.9 kb, pUC ori, kanamycin resistance, coding for human monocyte chemoattractant protein 1 under the control of the eucaryotic CMV promoter), batch fermentations with a semi-defined culture medium were carried out. This example shall demonstrate the superiority of JM108 compared to other host strains.

For preculture, a shake flask containing 200 mL medium, was inoculated with 1 mL of a glycerol stock of the respective strain and cultivated in a rotary shaker (37 °C, 300 rpm) until an optical density of 1-1.5 was obtained. The preculture medium consisted of 13.5 g/L soy peptone; 7 g/L yeast extract; 6 g/L glycerol; 2.5 g/L NaCl; 2.3 g/L $K_2HPO_4$; 1.5 g/L $KH_2PO_4$; and 0.25 g/L $MgSO_4$*$7H_2O$. The preculture was inoculated into the fermenters (1 % v/v of batch medium) which contained a medium of the following composition: 13.5 g/L soy peptone; 7 g/L yeast extract; 15 g/L glycerol; 0.5 g/L trisodium citrate; 1.2 g/L $KH_2PO_4$; 2.2 g/L $Na_2HPO_4$*$12H_2O$; 5 g/L $(NH_4)_2SO_4$; 4 g/L $NH_4Cl$; 0.8 g/L $MgSO_4$*$7H_2O$; 0.26 g/L $CaCl_2$*$2H_2O$; 1.5 ml/L trace element solution; and 5 mL/L thiamin hydrocloride solution (1%). The trace element solution contained: 27 g/L $FeCl_3$*$6H_2O$; 8 g/L $ZnSO_4$*$7H_2O$; 7 g/L $CoCl_2$*$6H_2O$; 7 g/L $MoNa_2O_4$*$2H_2O$; 8 g/L $CuSO_4$*$5H_2O$; 2 g/L $H_3BO_3$ ; and 5 g/L $MnSO_4$*$7H_2O$. The cultivation temperature was 37 °C and the pH was controlled at 7.0 with 25 % w/v NaOH and 25 % w/v $H_2SO_4$. A dissolved oxygen tension (DOT) of $\geq$ 20 % saturation was maintained with a constant aeration rate of 1 wm and if necessary, by the increase of the agitation rate (500-1000 rpm). The cultivations were terminated when the culture was in the stationary growth phase for 3 - 4 h, determined by measuring the optical density.

Table 1 shows the results of these fermentations. Although JM108 grew only to a low biomass, the volumetric plasmid yield was highest with 112 mg pDNA/L. This is an advantage for plasmid DNA production, since low amounts of biomass simplify large scale alkaline lysis during downstream processing. This reduced growth activity resulted in the extraordinary high specific pDNA yield of 8.2 mg/OD*L (which corresponds to approximately 30 mg pDNA/g dry cell weight).

Table1

| Results of growth, pDNA yield and pDNA homogeneity of batch fermentations of several *E. coli* host strains. | | | | | | | | | |
|---|---|---|---|---|---|---|---|---|---|
| *E. coli* host strain | DH1 | DH5 | DH5$\alpha$ | JM83 | JM101 | HB101 | XL1-blue | W3110 | JM108 |
| $OD_{550}$ max | 29 | 27 | 35 | 14 | 32 | 17 | 26 | 27 | 14 |
| Plasmid DNA [mg/L] | 87 | 102 | 46 | 29 | 64 | 69 | 105 | 23 | 112 |
| Spec. pDNA [mg/OD*L] | 3.0 | 3.8 | 1.3 | 2.1 | 2.0 | 4.1 | 4.0 | 0.9 | 8.2 |
| Homogeneity [% ccc] | 84 | 85 | 65 | 55 | 61 | 93 | 83 | 50 | 91 |

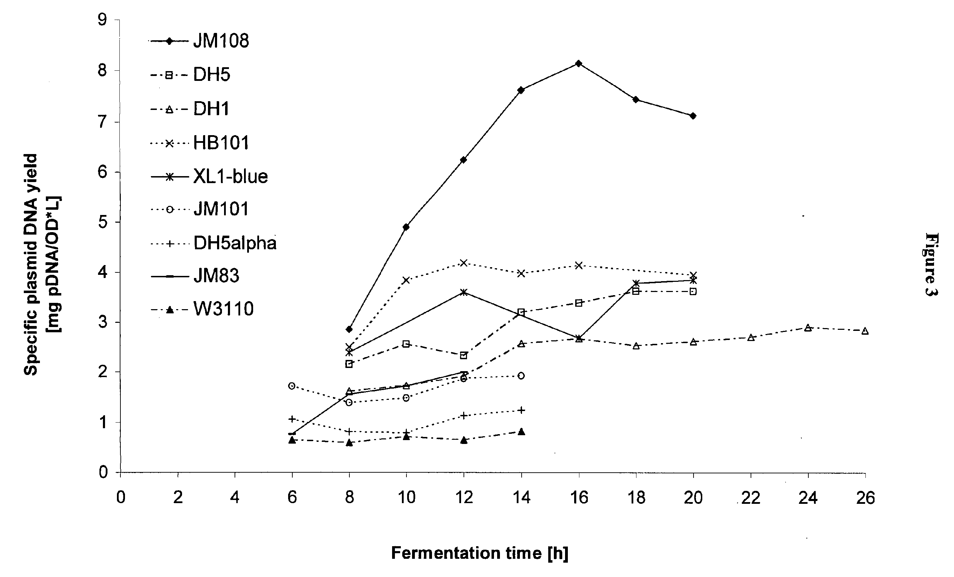

In Figure 3, the course of generation of specific pDNA of all strains is shown. The particular advantage of the use of the strain JM108 is an enhanced plasmid replication activity which exceeds the plasmid content in the biomass significantly compared to other strains. This surprising behavior of JM108 is specific to controlled fermentations and was not observed in shake flask cultivation. In addition, the percentage of supercoiled pDNA (ccc form) was over 90 % throughout the whole fermentation time. In combination with the exceptional yield data, this makes JM108 a superior host strain for pDNA production with a higher performance than other strains.

**Example 1**

Batch fermentation of *E. coli* JM108 (pRZ-hMCP1) in a semi-defined medium on different manufacturing scales

Fermentations were carried out on a manufacturing scale of 20 L and 200 L, in the same way as described in Example 2. It is shown in Table 2 that on both manufacturing scales, a high volumetric and specific yield was obtained. Moreover, pDNA homogeneity was consistently ≥ 90 % of supercoiled form. This example shows that the application of JM108 is not restricted to a certain fermentation scale.

Table 2

| Batch fermentations of *E. coli* JM108 (pRZ-hMCP1) with semi-defined medium on a manufacturing scale of 20 L and 200 L (data from end of fermentation). | | | | |
|---|---|---|---|---|
| Scale (volume culture broth) | 20 L #1 | 20 L #2 | 200 L #1 | 200 L #2 |
| Dry cell weight [g/L] | 4.0 | 3.4 | 3.8 | 4.0 |
| Plasmid DNA [mg/L] | 89 | 91 | 106 | 121 |
| Specific pDNA [mg/g DCW*L] | 23 | 27 | 28 | 30 |
| Homogeneity [% ccc] | 90 | 94 | 93 | 92 |

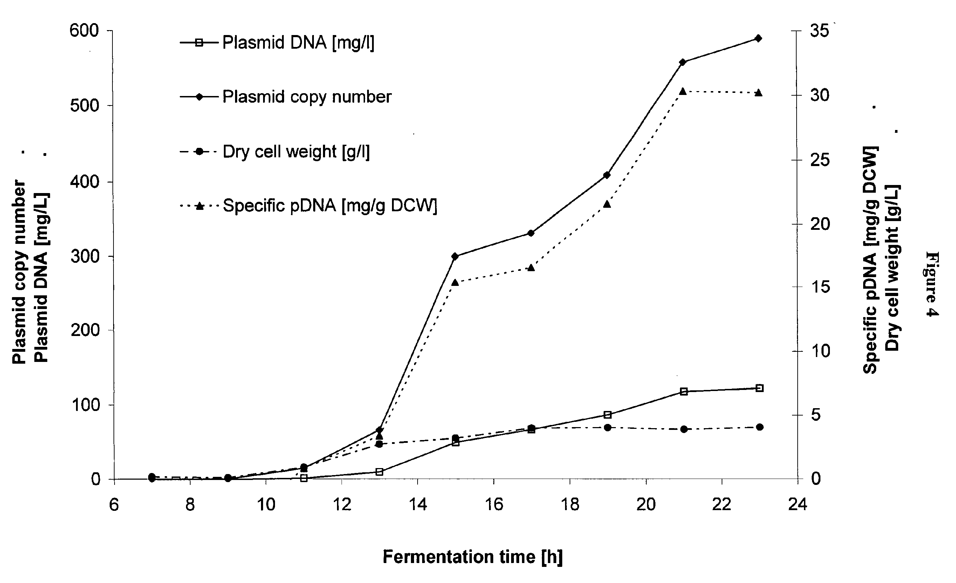

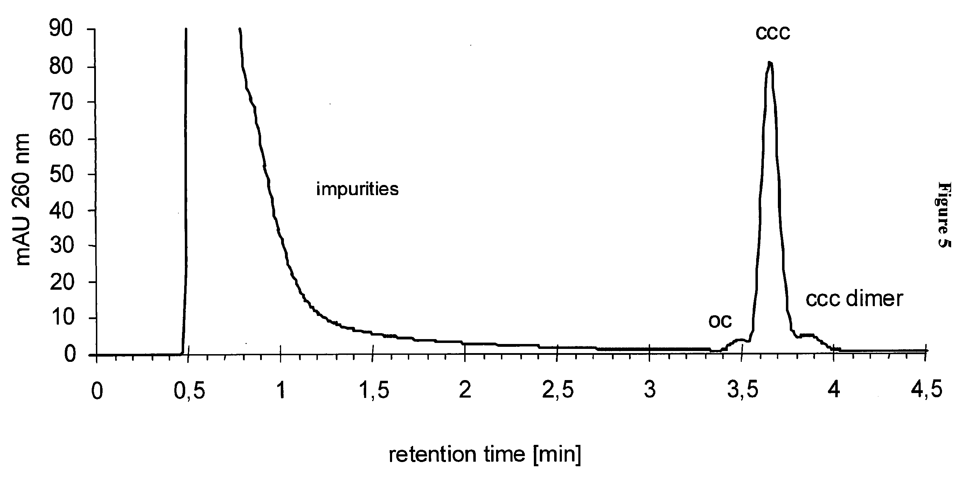

In Figure 4 the time course of the parameters of the fermentation 200L #2 are shown. The plasmid copy number, which is defined as the number of plasmid molecules per genome, increased up to 600 towards the fermentation end. This corresponded with the course of the specific pDNA. In Figure 5, an analytical chromatogram (anion exchange HPLC) is shown from the end of fermentation 200 #2, demonstrating high pDNA homogeneity.

**Preliminary Experiment 3**

Batch fermentation of *E. coli* JM108 harboring the plasmid pRZ-hMCP1 in a chemically defined medium

A batch fermentation was carried out in a 1 L scale fermenter with the *E. coli* JM108 harboring the plasmid pRZ-hMCP1. For a preculture, a glycerol stock of the strain (300 µL) was inoculated into a baffled 1000 mL shake flask containing 300 mL of a defined medium. This was cultivated in a rotary shaker at 300 rpm and 37 °C. The preculture medium was composed as follows: $NH_4Cl$ 2 g/L, $MgSO_4$*$7H_2O$ 0.24 g/L, glucose 10 g/L, L-proline 0.2 g/L, L-isoleucine 0.2 g/L, thiamine hydrochloride 1 mg/L, citric acid 2 g/L, $KH_2PO_4$ 5.44 g/L, $Na_2HPO_4$*$12H_2O$ 14.38 g/L and trace element solution 16.7 mL/L. The trace element solution contained HCl (25 %) 14.6 g/L, $CaCl_2$*$2H_2O$ 0.44 g/L, $FeSO_4$*$7H_2O$ 0.33 g/L, $CoCl_2$*$6H_2O$ 0.14 g/L, $MnSO_4$*$H_2O$ 0.10 g/L, $CuSO_4$*$5H_2O$ 15 mg/L and $ZnSO_4$*$7H_2O$ 17 mg/L. As the pre-culture has reached an optical density of approximately OD = 1, it was transferred into the fermenter and the fermentation was started. The main culture batch medium was composed of the same components with the same concentrations as present in the pre-culture. The temperature was controlled at 37 °C. The fermenter was aerated with a process air mass flow rate of 1 wm (volume air per volume medium and minute). When the dissolved oxygen tension dropped down to 30 %, it was maintained at this set point by increasing the agitation rate of the stirrer (500-1000 rpm). The pH was controlled at the set point of 7.0 with a solution of ammonium hydroxide (25 %) and 25 % $H_2SO_4$.
As the culture had entered the stationary growth phase, the cultivation was extended for additional 20 h to observe the course of plasmid replication and plasmid homogeneity.

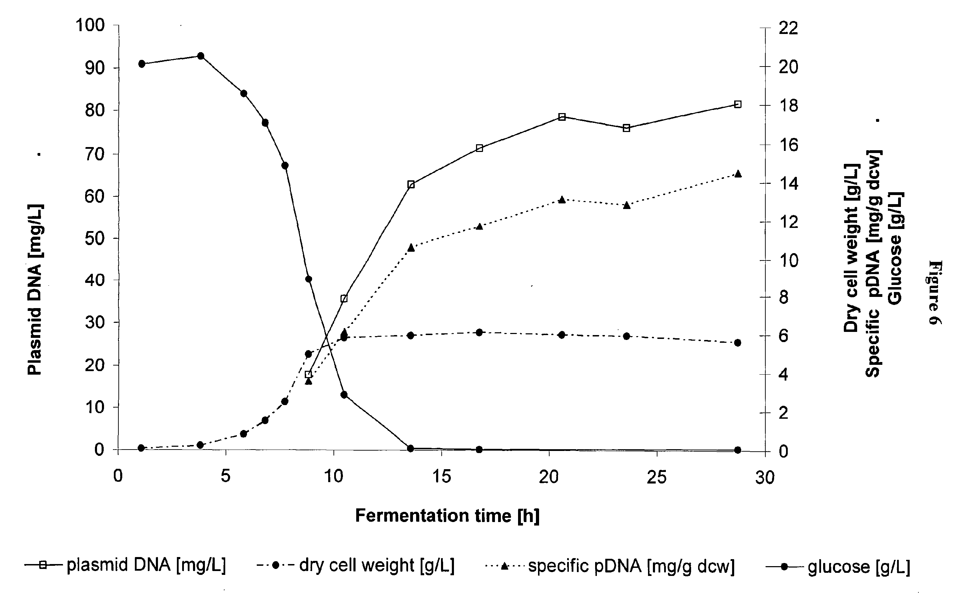

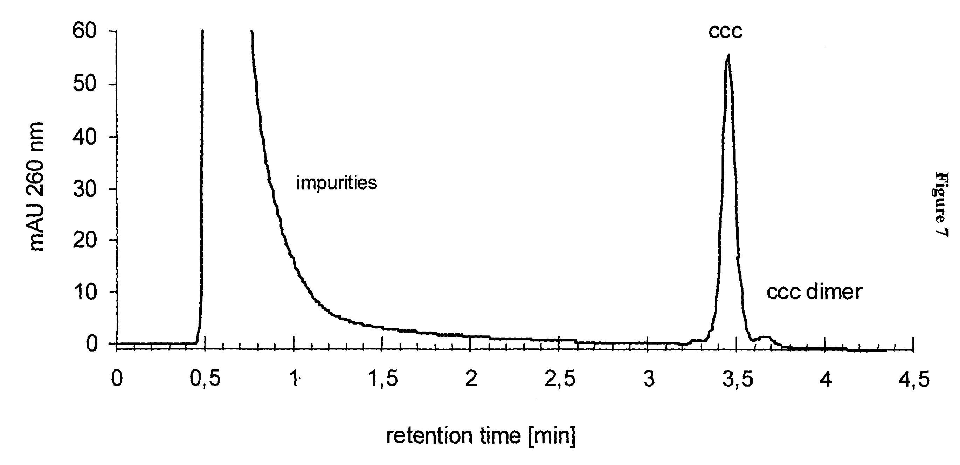

Figure 6 shows that even when growth is stopped upon glucose depletion, the plasmid yield (volumetric and specific) still increases. A pDNA volumetric yield of 82 mg/L was obtained, the specific yield was 14 mg pDNA/g dry cell weight at the end of the fermentation. In Figure 7, the excellent pDNA homogeneity of 95 % ccc form is demonstrated in this embodiment of the invention.

### Example 2

Fed-batch fermentation of *E. coli* JM108 (pRZ-hMCP1) in a semi-defined medium by applying an exponential feeding algorithm

A fed-batch fermentation was carried out by first cultivating the strain JM108 in a semi-defined batch medium as described in Preliminary Experiment 2, followed by feeding according to an exponential function upon the batch phase. The scale was 20 L culture broth volume at the end of the fermentation. The preculture and the batch cultivation phase were identical as in Example 2, with the same media composition and the same cultivation conditions. After 12 h of batch cultivation, the feed was initiated in order to control the specific growth rate μ at the value of 0.1 h-1. The volume of feeding medium to be added after a certain time point ($V_t$) was calculated according to the following function:

$$V_t = \frac{X_0}{Y_{X/-S^*}C_s} * e^{\mu t}$$

In this function, $V_t$ is the volume [L] of feeding medium to be added at the time distance t [h] calculated from the start of the feed. $X_0$ is the total amount of biomass dry cell weight [g] at the time point of start feed. $Y_{X/S}$ is the biomass yield coefficient (g dry cell weight per g substrate) and $C_S$ is the concentration of the substrate (glycerol as organic carbon source) in the feeding medium [g/L]. The specific growth rate μ [h-1] was set at the value of 0.1 h-1. The biomass yield coefficient was estimated as 0.25 g biomass per g glycerol. The biomass amount at the start of feeding $X_0$ was estimated by measurement of the optical density at the time point of start feeding.

The feeding medium was a concentrated solution of all necessary nutrients and was composed as follows: 83 g/L soy peptone; 42 g/L yeast extract; 250 g/L glycerol; 1.4 g/L trisodium citrate; 3.4 g/L $KH_2PO_4$; 6.2 g/L $Na_2HPO_4*12H_2O$; 14.2 g/L $(NH_4)_2SO_4$; 11.3 g/L $NH_4C$); 2.3 g/L $MgSO_4*7H_2O$; 0.7 g/L $CaCl_2*2H_2O$; 14.2 mL/L thiamin hydrocloride solution (1 %) and 4.3 ml/L trace element solution, composed as described in Example 2.

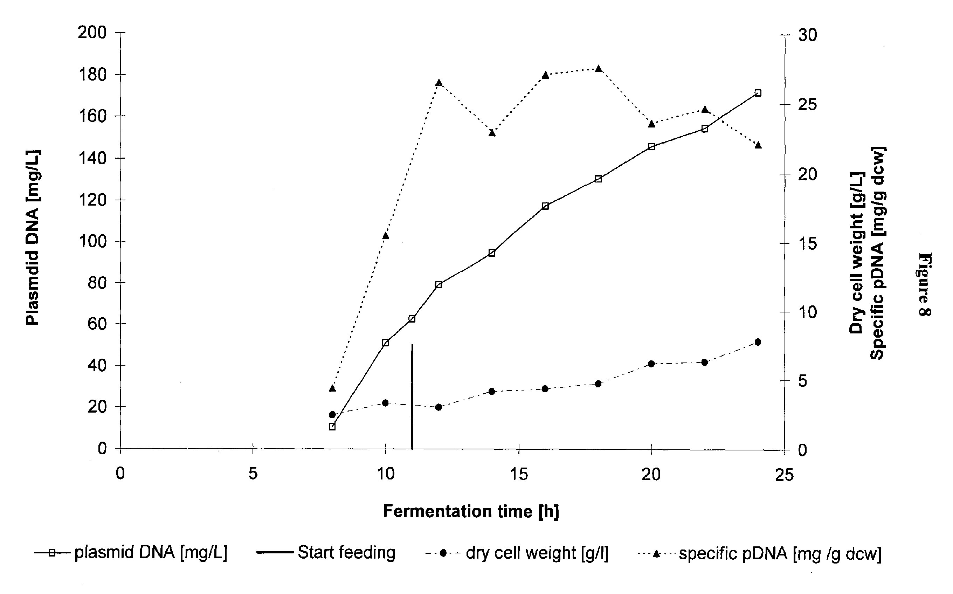

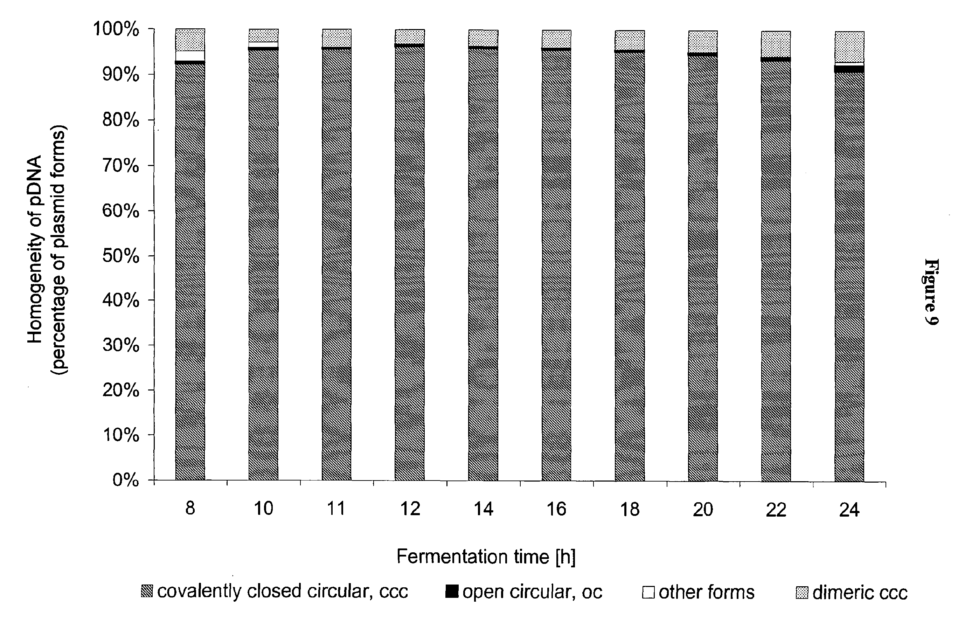

As shown in Figure 8, the specific pDNA concentration increased up to more than 20 mg pDNA per g dry cell weight during the batch cultivation phase. Upon initiation of feeding at a dry cell weight of 3 g/L (corresponding to an optical density of $OD_{550}$ = 13), this high specific pDNA yield was maintained until the end of the fermentation. At this time point, the volumetric pDNA yield had increased up to 170 mg/L. This example shows that JM108 is able to replicate plasmids to a high volumetric and specific pDNA yield without growing to a high cell density. This has the advantage that less biomass has to be processed during alkaline lysis and the following purification steps. Figure 9 demonstrates the advantage of JM108 in maintaining a high plasmid homogeneity throughout the whole fermentation process at the value of more than 90 % supercoiled pDNA.

### Example 3

Fed-batch fermentation of *E. coli* JM108 carrying the plasmid pRZ-hMCP1 (20 L fermenter) by using a chemically defined culture medium and an exponential feeding algorithm

An exponential fed-batch fermentation was carried out in a 20 L scale fermenter with *E. coli* JM 108 harboring the plasmid pRZ-hMCP1 in a process using a chemically defined culture medium (i. e. without complex medium compounds).

For a pre-culture, a glycerol stock of the strain (300 μL) was inoculated into a baffled 1000 mL shake flask containing 300 mL of a defined medium. This was cultivated in a rotary shaker at 300 rpm and 37 °C. The preculture medium was composed as described in Example 4. When the preculture had reached an optical density of approximately $OD_{550}$ = 1, it was transferred into the fermenter and the fermentation was started. The main culture batch medium was composed of the same components with the same concentrations as present in the preculture. The fermenter contained 7 L of batch medium at the onset of the fermentation. The temperature was controlled at 37 °C and the fermentation was operated with a back pressure of 0.35 bar. The fermenter was aerated with a process air mass flow rate of 1 wm (volume air per volume medium and minute = 7 L/min). When the dissolved oxygen tension dropped down to 30 %, it was maintained at this set point by increasing the agitation rate of the stirrer (500-1000 rpm). In case the increase of the agitation rate was not sufficient to maintain the DO, the oxygen concentration of the air was enriched with pure oxygen. The pH was controlled at the set point of $7.0 \pm 0.2$ with a solution of ammonium hydroxide (25 %), which concomitantly served as source of nitrogen throughout the fermentation. If necessary, the pH was further controlled with 25 % $H_2SO_4$.

After 10 h of batch cultivation, glucose in the batch medium was used up. This was determined with a rapid off-line measurement method (Yellow Springs Glucose Analyzer, YSI 2700 Select). The depletion of glucose served as the signal for the start of the exponential feeding phase. Continuous exponential feeding was controlled via the process control system of the fermenter, based on biomass at the time point of glucose depletion (estimated via optical density). The feeding medium was composed as follows: glucose 300 g/L; $MgSO_4*7H_2O$ 7.2 g/L; L-proline 6 g/L; L-isoleucine 6 g/L; thiamine hydrochloride 30 mg/L; citric acid 2 g/L; $KH_2PO_4$ 5.4 g/L; $Na_2HPO_4*12H_2O$ 14.4 g/L; $CaCl_2*2H_2O$ 220 mg/L; $FeSO_4*7H_2O$ 170 mg/L; $CoCl_2*6H_2O$ 72 mg/L, $MnSO_4*H_2O$ 51 mg/L, $CuSO_4*5H_2O$ 8 mg/L and $ZnSO_4*7H_2O$ 9 mg/L. The feeding rate was chosen to obtain a pre-defined specific growth rate $\mu$ of 0.1 h$^{-1}$. The calculation of the feeding rate was accomplished in a similar way as described in Example 5.

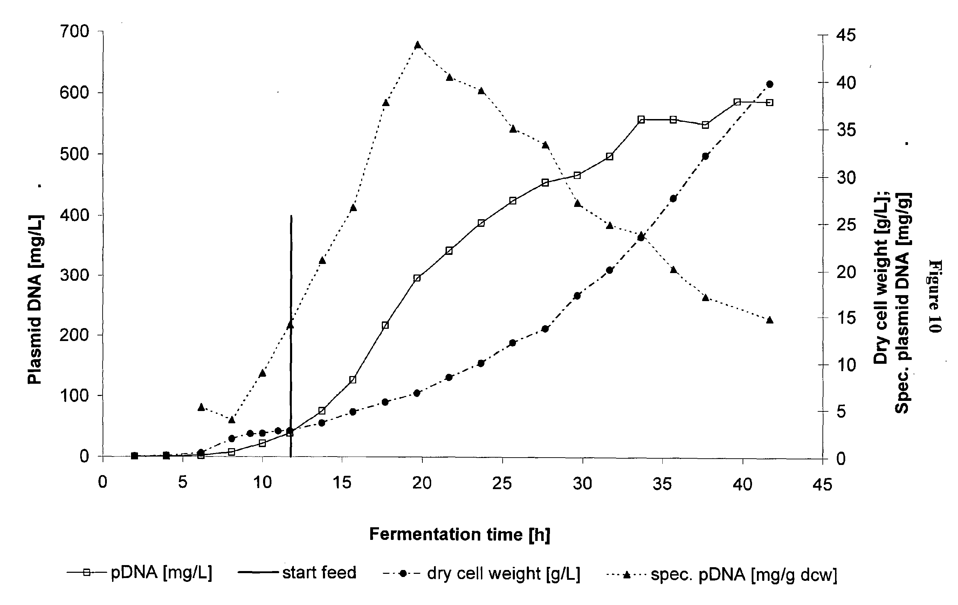

In Figure 10, the volumetric and specific plasmid yield and the growth curve are shown. At the end of the fermentation, an exceptionally high volumetric pDNA yield of 590 mg/L was obtained. In addition, at 20 h of fermentation time, the specific pDNA yield reached a maximum of 44 mg pDNA per g dry cell weight, which is also an exceptionally high value. Towards the fermentation end, the specific yield decreased down to 15 mg/ g DCW. The advantage of this characteristic course of the specific and volumetric yield is that several options for the termination of the fermentation can be chosen. If biomass with the highest content of pDNA must be obtained, the fermentation can be terminated after 20 h, resulting in a volumetric yield of 300 mg/L. If the process goal is the highest volumetric yield, the fermentation can be prolonged beyond 40 h, which results in about 600 mg/L, but a lower specific yield. The plasmid homogeneity of this fermentation was maintained at > 89 % ccc towards the end of the fermentation.

This example shows that the strain JM108 is superior compared to state-of-the-art host strains in terms of pDNA yield and homogeneity, in particular when a defined medium is used, in combination with exponential feeding.

**Example 4**

Comparison of state-of-the-art *E. coli* host strains with the strain JM108 in plasmid fermentation

In Table 3, data obtained from the fermentations described in Examples 1 to 3 were compared between prior-art strains and JM108. It could be shown that, in all fermentation processes, JM 108 is superior to the prior-art strains in terms of volumetric and specific yield. In addition, the pDNA homogeneity obtained with JM108 never was below 89 % ccc.

Table 3

| Comparison of state-of-the-art *E. coli* host strains with JM108 in fermentations for producing plasmid DNA. | | | | | | | |
|---|---|---|---|---|---|---|---|
| **Strain** | **cult type*)** | **medium **)** | **biomass** | **volumetr. pDNA yield max. [mg/L]** | **spec. pDNA yield max.** | **pDNA homogeneity [%ccc]** | **Reference** |
| DH10 B | B | SD | 4.8 g/L | 49.3 | 10.3 mg/g DCW | - | Lahijani et al. 1996 |
| DH10 B | FB | SD | - | 218.6 | 2.8 mg/ OD*L | - | Lahjani et al.1996 |
| DH10 B | FB | SD | 60 g/L | 100 | 1.7 mg/g DCW | - | Chen et al 1997 |
| DH5$\alpha$ | B | SD | 2.6 g/L | 8.5 | 0.5 mg/ OD*L 3.3 mg/g DCW | 50 % ccc | O'Kennedy et al. 2003 |
| DH5$\alpha$ | FB | SD | 3.4 g/L | 25.6 | 1.8 mg/ OD*L 7.6 mg/g DCW | 70 % ccc | O'Kennedy et al. 2003 |
| DH5$\alpha$ | B | D | 22 g/L | 68 | 3.1 mg/g DCW | 97% ccc | WO 02/064752 |
| DH5$\alpha$ | SF | SD | 2.5 g/L | 7 | 2.8 mg/g DCW | - | O'Kennedy et al.2000 |
| DH5$\alpha$ | FB | SD | 48 g/L | 200 | 4.2 mg/g DCW | > 90 % ccc | WO 99/61633 |

*) cultivation type: SF...shake flask cultivation, B...batch fermentation; FB...fed-batch fermentation

**) medium: D...chemically defined, synthetic; SD...semi-defined; C...complex

Table 3   (continued)

| Strain | cult type*) | medium **) | biomass | volumetr. pDNA yield max. [mg/L] | spec. pDNA yield max. | pDNA homogeneity [%ccc] | Reference |
|---|---|---|---|---|---|---|---|
| Comparison of state-of-the-art *E. coli* host strains with JM108 in fermentations for producing plasmid DNA. | | | | | | | |
| DH5α | FB | D | 48 g/L | 100 | 2.1 mg/g DCW | - | WO 99/61633 |
| DH5α | FB | SD | 60 g/L | 230 | 3.8 mg/g DCW | > 90 % ccc | WO 99/61633 |
| CP79 | B | D | 0.64 g/L | 2.1 | 3.3 | - | Hofmann et al. 1990 |
| CP79 | FB | D | 5 g/L | 19.8 | 3.9 | - | Hofmann et al.1990 |
| CP143 | B | D | 0.28 g/L | 1.5 | 5.4 | - | Hofmann et al. 1990 |
| CP143 | B | D | 6.8 g/L | 47.8 | 7 | - | Hofmann et al.1990 |
| HB101 | B | C | - | 4.5 | - | - | Renikainen et al. 1989 |
| XAC-1*pir11* 6 | FB | SD | 44 g/L | 115 | 2.6 mg/g DCW | - | Soubrier et al. 1999 |
| XAC-1*pir11* 6 | FB | D | 30 g/L | 100 | 3.3 mg/g DCW | - | Soubrier et al. 1999 |
| JM108 | B | SD | 3.4-4.0 g/L | 89-121 | 23-30 mg/g DCW | 90-94 % ccc | |
| JM108 | B | D | 5.8 g/L | 82 | 14 | 95 % ccc | |
| JM108 | FB | SD | 8 g/L | 170 | > 20 mg/g DCW | > 90 % ccc | |
| JM108 | FB | D | 40 g/L | 600 | 15-45 mg/g DCW | > 89 % ccc | |

*) cultivation type: SF...shake flask cultivation, B...batch fermentation; FB...fed-batch fermentation

**) medium: D...chemically defined, synthetic; SD...semi-defined; C...complex

References

Atlung, T., Christensen, B.B., and Hansen, F.G. (1999). Role of the Rom protein in copy number control of plasmid pBR322 at different growth rates in *Escherichia coli* K-12. Plasmid 41, 110-119.

Birnboim, H.C. and Doly, J. (1979). A rapid alkaline extraction procedure for screening recombinant plasmid-DNA. Nucl. Acids Res. 7, 1513-1523.

CBER, Center for Biologics Evaluation and Research, U.S. Food and Drug Administration, Rockville, MD (1996). Points to consider on plasmid DNA vaccines for preventive infectious disease indications.

Chen, W., Graham, C., and Ciccarelli, R.B. (1997). Automated fed-batch fermentation with feed-back controls based on dissolved oxygen (DO) and pH for production of DNA vaccines. J. Ind. Microbiol. Biotechnol., 18, 43-48.

Durland, R.H. and Eastman, E.M. (1998). Manufacturing and quality control of plasmid-based gene expression systems. Adv. Drug. Del. Rev. 30, 33-48.

EMEA, European Agency for the Evaluation of Medicinal Products (2001). *Note for Guidance on the Quality, Preclinical and Clinical Aspects of Gene Transfer Medicinal Products;* CPMP/BWP/3088/99.

Furutani Y., Nomura H., Notake M., Oyamada Y., Fukui T., Yamada M., Larsen C. G., Oppenheim J. J. and Matsushima K. (1989). Cloning and sequencing of the cDNA for human monocyte chemotactic factor (MCAF). Biochem Biophys Res Commun 159(1), 249-55.

Herman, R.E. and McKay, L.L. (1986). Cloning and expression of the β-D-galactosidase gene from *Strepto-*

*coccus thermophilus* in *Escherichia coli.* Appl. Env. Microbiol. 52/1, 45-50.

Hofmann, K.H., Neubauer, P., Riethdorf, S., and Hecker, M. (1989). Amplification of pBR322 plasmid DNA in *Escherichia coli relA* strains during batch and fed-batch fermentation. J. Basic Microbiol. 30, 37-41.

Ikeda, H., Nonomiya, T., Usami, M., Ohta, T. and Omura, S. (1999). Organization of the biosynthetic gene cluster for the polyketide anthelminthic macrolide avermectin in *Streptomyces avermilitis.* Proc. Natl. Acad. Sci. 96 (17) 9509-9514.

Kakinuma, S., Takada, Y., Ikeda, H., Tanaka, H., Omura, S., and Hopwood, D.A. (1991). Cloning of large DNA fragments, which hybridize with actinorhodin biosynthesis genes, from kalafungin and nanaomycin a methyl ester producers and identification of genes for kalafungin biosynthesis of the kalafungin producer. J. Antibiotics 44/9, 995-1005.

Lahijani, R., Hulley, G., Soriano, G., Horn, N.A., and Marquet, M. (1996). High-yield production of pBR322-derived plasmids intended for human gene therapy by employing a temperature-controllable point mutation. Hum. Gene Ther. 7, 1971-1980.

O'Kennedy, R.D., Baldwin, C., and Keshavarz-Moore, E., (2000). Effects of growth medium selection on plasmid DNA production and initial processing steps. J. Biotechnol. 76, 175-183.

O'Kennedy et al (2003). Effects of fermentation strategy on the characteristics of plasmid DNA production. Biotechnol Appl. Biochem. 34, 83-90.

Omura, S., Ikeda, H., Ishikawa, J., Hanamoto, A., Takahashi, C., Shinose, M., Takahashi, Y., Horikawa, H., Nakazawa, H., Osonoe, Kikuchi, H., Shiba, T., Sakaki., Y., and Hattori, M. (2001). Genome sequence of an industrial microorganism *Streptomyces avermitilis:* deducing the ability of producing secondary metabolites. Proc. Natl. Acad. Sci. 98 (21) 12215-12220.

Reinikainen, P., Korpela, K., Nissinen, V., Olkku, J., Söderlund, H., and Markkanen, P. (1988). *Escherichia coli* plasmid production in Fermenter. Biotech. Bioeng. 33, 386-393.

Riegert, U., Heiss, G., Fischer, P., and Stolz, A. (1998). Distal cleavage of 3-chlorocatechol by an extradiol dioxygenase to 3-chloro-2-hydroxymuconic semialdehyde. J. Bacteriology 180/11, 2849-2853.

Sambrook, J., and Russel, D.W. (2001). *Molecular Cloning - a Laboratory Manual,* 3rd ed., J. Argentine, N. Irwin, K.A. Janssen, S. Curtis, M. Zierler, et. al., eds. (Cold Spring Harbor, NY).

Schoenfeld, T., Mendez, J., Storts, D.R., Portman, E., Patterson, B., Frederiksen, J., and Smith, C. (1995). Effects of bacterial strains carrying the *endA1* genotype on DNA quality isolated with Wizard Plasmid Purification System. Promega Notes, 53, 12 - 22.

Schleef, M. (1999). Issues of large-scale plasmid DNA manufacturing. In *Biotechnology,* 2nd ed. Vol. 5a., H. J Fehm and G. Reed, eds. (Wiley-VCH, Weinheim), pp. 443-469.

Soubrier, F., Cameron, B., Manse, B., Somarriba, S. Dubertret, C., Jaslin, G., Jung, G., LE Caer, C., Dang, D., Mouvault, J.M., Scherman, D., Mayaux, J.F., and Crouzet, J. (1999). pCOR: a new design of plasmid vectors for nonviral gene therapy. Gene Ther. 6, 1482-1488.

Taylor, R.G., Walker, D.C., and Mdnnes, R.R. (1993). E. *coli* host strains significantly affect the quality of small scale plasmid DNA preparations used for sequencing. Nucleic Acids Res. 21, 1677-1678.

WHO (1998). Guidelines for assuring the quality of DNA Vaccines. WHO Technical Report Series 878, 77-90.

Wrobel, B. and Wegrzyn, G. (1998). Replication regulation of ColE1-like plasmids in amino acid-starved *Escherichia coli*. Plasmid 39, 48-62.

Yanisch-Perron, C., Vieira, J., and Messing, J. (1985). Improved M13 phage cloning vectors and host strains: nucleotide sequences of the M13mp18 and pUC19 vectors. Gene 33, 103-119.

## Claims

1.    A method for producing plasmid DNA on a manufacturing scale by fermenting *Escherichia coli* cells that contain a plasmid carrying a gene of interest, disintegrating the cells and isolating and purifying the obtained plasmid DNA, wherein said cells are cells of *Escherichia coli* K-12 strain JM108 or a derivative of said strain.

2.    The method of claim 1, wherein said cells are fermented in a batch mode.

3.    The method of claim 1, wherein said cells are fermented in a fed-batch mode.

4.    The method of claim 1, wherein said cells are fermented in a semi-defined culture medium.

5.    The method of claim 1, wherein said cells are fermented in a chemically defined culture medium.

**6.** The method of claim 1, wherein said cells contain a plasmid with a ColE1 origin of replication.

**7.** The method of claim 6, wherein said plasmid is a pUC type plasmid.

# Figure 1

## Figure 2

**Figure 3**

**Figure 4**

Figure 5

# Figure 6

Figure 7

# Figure 8

Figure 9

Figure 10

EP 1 584 687 A1

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DE 101 06 493 A (PLASMIDFACTORY GMBH & CO KG) 14 August 2002 (2002-08-14) * column 5, paragraph 31; figure 7; examples 1-5 * --- | 1-7 | C12N15/69 |
| X | O'KENNEDY RONAN D ET AL: "Effects of fermentation strategy on the characteristics of plasmid DNA production." BIOTECHNOLOGY AND APPLIED BIOCHEMISTRY, vol. 37, no. 1, February 2003 (2003-02), pages 83-90, XP002283747 ISSN: 0885-4513 * page 84; figure 4 * --- | 1-7 | |
| A | YANISCH-PERRON C ET AL: "IMPROVED M13 PHAGE CLONING VECTORS AND HOST STRAINS: NUCLEOTIDE SEQUENCES OF TH EM13MP18 AND PUC19 VECTORS" GENE, ELSEVIER BIOMEDICAL PRESS. AMSTERDAM, NL, vol. 33, 1985, pages 103-119, XP002060738 ISSN: 0378-1119 * the whole document * --- | | |
| A | HERMAN R E ET AL: "CLONING AND EXPRESSION OF THE BETA-D GALACTOSIDASE GENE FROM STREPTOCOCCUS-THERMOPHILUS IN ESCHERICHIA-COLI" APPLIED AND ENVIRONMENTAL MICROBIOLOGY, vol. 52, no. 1, 1986, pages 45-50, XP002283748 ISSN: 0099-2240 * the whole document * ----- | | |

**TECHNICAL FIELDS SEARCHED (Int.Cl.7)**

C12N

The present search report has been drawn up for all claims

| Place of search | Date of completion of the search | Examiner |
|---|---|---|
| MUNICH | 9 June 2004 | Trommsdorff, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**EP 1 584 687 A1**

**ANNEX TO THE EUROPEAN SEARCH REPORT
ON EUROPEAN PATENT APPLICATION NO.**

EP 04 00 8557

This annex lists the patent family members relating to the patent documents cited in  the above-mentioned European search report.
The members are as contained in the European Patent Office EDP file on
The European Patent Office is in no way liable for these particulars which are merely  given for the purpose of information.

09-06-2004

| Patent document cited in search report | Publication date | Patent family member(s) | Publication date |
|---|---|---|---|
| DE 10106493         A | 14-08-2002 | DE    10106493 A1<br>WO    02064752 A1 | 14-08-2002<br>22-08-2002 |

EPO FORM P0459

For more details about this annex : see Official Journal of the European Patent Office, No. 12/82

24